(19) Europäisches Patentamt
European Patent Office
Office européen des brevets

(11) **EP 2 121 129 B1**

(12) **EUROPEAN PATENT SPECIFICATION**

(45) Date of publication and mention
of the grant of the patent:
**12.01.2011 Bulletin 2011/02**

(51) Int Cl.:
***A61N 5/00*** *(2006.01)*     ***A61M 36/00*** *(2006.01)*

(21) Application number: **08719963.4**

(86) International application number:
**PCT/IL2008/000338**

(22) Date of filing: **12.03.2008**

(87) International publication number:
**WO 2008/111065 (18.09.2008 Gazette 2008/38)**

(54) **THULIUM-CONTAINING LOW DOSE RATE BRACHYTHERAPY SEED**

THULIUM ENTHALTENDES BRACHYTHERAPIE-SEED MIT NIEDRIGER DOSISRATE

GRAIN DE CURIETHÉRAPIE À FAIBLE DOSE CONTENANT DU THULIUM

(84) Designated Contracting States:
**AT BE BG CH CY CZ DE DK EE ES FI FR GB GR
HR HU IE IS IT LI LT LU LV MC MT NL NO PL PT
RO SE SI SK TR**

(30) Priority: **12.03.2007 US 906204 P**

(43) Date of publication of application:
**25.11.2009 Bulletin 2009/48**

(73) Proprietor: **Ben-Gurion University Of The Negev
Research
And Development Authority
84105 Beer-Sheva (IL)**

(72) Inventors:
• **SHANI, Gad**
  **84965 Omer (IL)**
• **AYOUB, Amal**
  **84719 Be'er Sheva (IL)**

(74) Representative: **Turner, Craig Robert
A.A. Thornton & Co.
235 High Holborn
London WC1V 7LE (GB)**

(56) References cited:
EP-A- 0 960 633     EP-A- 1 084 733
EP-A- 1 232 770     WO-A-98/30273
WO-A-03/063757     US-A1- 2006 219 956

**Description**

Brachytherapy is a cancer treatment method in which ionizing radiation source is inserted into, or adjacent, a cancerous tumor, in order to kill cancer cells or shrink tumors. There are two common Brachytherapy treatment types: i) High Dose Rate (HDR); and ii) Low Dose Rate (LDR), which are distinct from one another by the intensity of the radiation source, its form and its method of use.

In LDR brachytherapy, low activity sources (also referred to as seeds or capsules), having diameter of about 0.8 mm and length of about 4.5 mm, are inserted into the tumor for a long period of time, practically till the activity of the radiation source decays completely. The activity of the LDR seeds is of the order of 0.5 millicurie (mCi). In order to obtain the radiation dose needed for killing all malignant cells in the tumor, a large number of seeds (100-120) are simultaneously implanted into the tumor.

Low dose rate brachytherapy is more or less limited to prostate cancer treatment while HDR brachytherapy is applied to other kinds of cancer too. Current LDR brachytherapy is also limited to tumors smaller than 50 cc, because of the large number of seeds required.

At present LDR brachytherapy is carried out using seeds containing the isotopes I 125 or Pd 103. I 125 emits photons of energy of about 28 keV and has a half life of 60 days. Pd 103 emits photons of energy of about 20 keV and has a half life of 17 days. It should be noted that the production of I 125 and Pd 103 is complicated, requiring the use of accelerator irradiation, isotope separation and hot laboratories for carrying out the radiochemistry of the seed production.

It has been proposed that thulium 170 may be used in brachytherapy. For example, WO 03/063757 describes a low dose brachytherapy seed, comprising the radioactive isotope thulium 170 placed within a titanium canister.

EP 1529554 describes a radioactive radiation source for ophthalmic brachytherapy. The radiation emitting element, which is preferably selected from the group consisting of Y-90, TI-204, P-32 and Tm-170, is placed within a suitable tubular casing together with a shielding section made of metallic, ceramic or a plastic material. Among the metals proposed for preparing the shielding section, Pt, Pd, Au, Ag, Ir, Pb and W, together with their alloys, are specifically listed. The publication exemplifies the preparation of a radiation source comprising an oxide of yttrium-90 dispersed within an aluminum matrix, placed in a first metallic cylinder made of stainless steel. The first, stainless steel cylinder is inserted into a second cylinder made from Pt/Ir alloy. The structure thus obtained is finally sealed within a titanium cover. International patent publication No. WO2008/0-41230, published after the filing date of this patent, discloses a capsule for high dose rate brachytherapy, wherein the capsule comprises within its interior space thulium-170, and further comprises at least one layer of a radiation emission modifying metal, and specifically gold, wherein said layer is provided either internally within the capsule or on the outer surface thereof. The activity of the radiation source within the seed is not less than 3 curie.

U.S. patent application No. 2006/219956 describes a brachytherapy device having a converter material and a radioactive beta-emitter, wherein the emitted beta radiation is converted by the converter material into characteristic radiation.

The inventor has found that the isotope thulium 170 (Tm-170), may be effectively used as a radiation source for low dose rate brachytherapy, in combination with a radiation emission modifying metal, which is preferably selected from the group consisting of gold (Au) and platinum (Pt). It has been found that the radiation emitted from thulium 170, which is composed of beta radiation (electrons) and gamma radiation (photons), may be favorably modified by coating the thulium 170 with one or more of the heavy metals listed above, and more specifically with gold.

According to the present invention, the radiation source includes a radioactive section, which comprises thulium 170, and at least one layer of a metal coating (specifically a gold coating) provided on the radioactive section. Although the primary purpose served by the heavy metal coating is to reduce the intensity of the beta rays emission from the thulium, the metal coating concurrently increases the intensity of the gamma radiation emitted by the source through two distinct effects: a braking radiation (bremsstrahlung) effect in the metal coating, in which a part of the beta radiation turns into photons, and in addition, photons are also emitted from the metal coating in the form of characteristic x-rays, in response to absorption of some of the thulium 84 keV gamma rays. These effects are described in detail below.

Accordingly, by appropriately adjusting the thickness of the metal coating onto the thulium, it is possible control the intensities of the beta and gamma radiations emitted therefrom, generating a Tm-170 based low dose rate brachytherapy radiation source, **characterized in that**:

(i) the level of beta radiation emitted therefrom is reduced in comparison with that generated by Tm-170;
(ii) the photon emission spectrum of said source displays a lower ratio between the intensities of the characteristic peaks at 84 keV and 52 keV energy levels, relative to the ratio observed in the corresponding spectrum of Tm-170;
(iii) the photon emission spectrum of said source exhibits one or more peaks in the region between 65 keV and 84 keV energy levels indicative of photons emitted by the layer(s) of the radiation emission modifying metal;
(iv) the photon emission spectrum of said source comprises in addition to the discrete peaks also a continuous region indicative of the emission of braking radiation associated with the layer(s) of the radiation emission modifying metal.

The present invention thus primarily relates to a low dose rate brachytherapy seed, which comprises thulium-170 placed within a casing, wherein said seed further comprises at least one layer of a radiation emission modifying metal, said layer being provided either internally within the casing or on the outer surface thereof. The activity of the seed is not more than 3 millicurie, and preferably between 0.5 and 2.5 millicurie.

The low dose rate brachytherapy seed of the invention comprises a casing or shell for containing a radioactive source. The casing may be made from any human tissue - compatible metal having a low atomic weight, such as titanium or stainless steel. Titanium or alloys thereof are especially preferred. The casing is preferably in the form of a cylindrical canister having the following characteristic dimensions: a length in the range between 4 and 7 mm, preferably about 4.5 mm; an outer diameter of about 0.5-1.0 mm, preferably about 0.8 mm; and an inner diameter of about 0.3-0.9 mm, preferably about 0.7 mm, such that the thickness of the wall of the cylindrical canister is about 0.05mm. A relatively thin wall is important for the utilization of the beta rays generated by the thulium isotope. A thicker wall will absorb a certain percent of the beta rays. The outer diameter of the seed and its length as set forth above are as acceptable for LDR brachytherapy. The metal of which the canister is made is of course different from the radiation emission modifying metal to be applied in accordance with the invention. Hereinafter, the terms titanium casing, titanium canister or titanium capsule are used interchangeably.

Within the interior of the casing, the thulium 170 segment is placed. The seed preferably comprises a gold or platinum coating which at least partially, and preferably entirely, surrounds the thulium 170 segment, said coating being either interposed between the radioactive isotope and the inner walls of the casing, or alternatively, applied onto the outer faces of the casing.

The seed of the present invention may be produced, for example, by coating the natural thulium 169 with one or more layers of a radiation emission modifying metal (e.g., gold), placing the coated thulium 169 in a casing as described above, e.g., a titanium casing, sealing the casing and subjecting the same to a neutron activation in a nuclear reactor in order to convert the thulium 169 into the radioactive isotope thulium 170 with the desired activity. Alternatively, the radiation emission modifying metal coating is applied onto the outer faces the casing, either prior to or following the activation of the seed in a nuclear reactor. The activation parameters for transforming the natural thulium 169 into thulium 170 are set forth below. The metal coating, and especially the gold coating, is applied directly onto the thulium, or onto the outer surface of the casing, by means of various techniques. The thickness of the radiation emission modifying metal coating is preferably between a few atomic layers and up to 0.15 mm, and more preferably in the range between 0.02 and 0.1 mm. The thickness of the coating may be specifically tailored in order to adjust the radiation profile of the seed (namely, to determine the proportions of the beta and gamma radiation generated by the seed). One possible technique is electroplating, wherein the thulium is immersed in a solution containing gold ions and an electric current is passed through the solution to deposit a gold coating onto a thulium piece. Electroplating conditions (suitable gold solutions, medium stirring rates and current densities required for obtaining desired gold coatings) are known in the art. A second method is by gold evaporation.
Heat is applied to a gold wire or rod (generally by electric current). Due to the high temperature gold atoms are evaporated and deposited on the thulium.

A further technique for making the gold coating is known as "sputtering", and has been found to be especially useful for forming relatively thin gold coatings onto the thulium (from 10 nm and up to 1 $\mu$m). A gold electrode and the thulium piece to be coated are positioned in a closed chamber and are separated by a distance in the range of 3 and 8 cm. The chamber is maintained under argon atmosphere at a low, constant pressure. A voltage of the order of 1-2 kV is applied between a gold electrode (directly connected to a power source) and the thulium piece to be coated. The electric field generated in the chamber ionizes the gas and causes a current to flow into the gold electrode. This results in gold being sputtered from the electrode and being deposit onto the thulium piece. The thickness of the gold coating thus formed onto the thulium is given by the following equation:

$$\texttt{Gold Thickness [Å] = k} \bullet \texttt{I} \bullet \texttt{t}$$

Where I is the chamber current in mA, t is the spattering time in minutes and k is a constant characteristic of the device employed for the spattering.

The simplest technique for forming a gold coating is by wrapping the thulium piece with one or more gold foils of a desired thickness. Thin gold foils of various thicknesses are commercially available (Goodfellow, England).

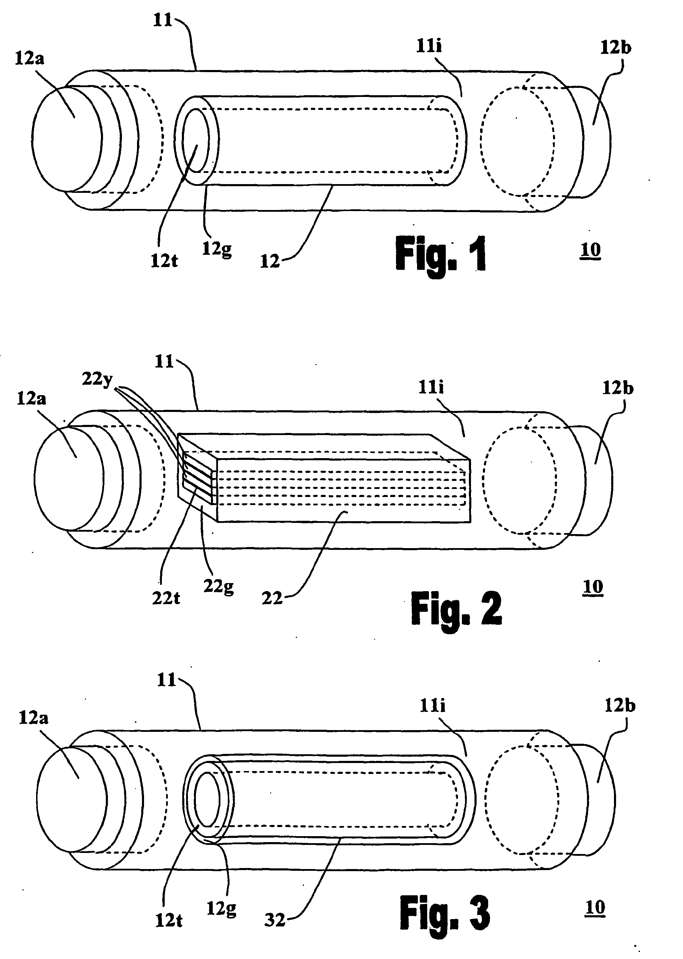

The amount of thulium placed in the seed according to the invention is preferably not less than 2 mg, and more preferably in the range between 2 and 8 mg. It is noted that thulium 169 to be converted according to the present invention to the radioactive thulium 170 is a readily processed metal, such that the final radioactive segment within the seed may attain various desired geometries, as illustrated in Figures 1, 2 and 3.

With reference to Figure 1, a low dose brachytherapy seed 10 comprises a titanium tube 11 having the dimensions set forth above. The natural thulium 169 is commercially available in the form of a wire of a small diameter, typically about 0.45-0.65 mm. A piece of the wire, having length of about 1.0-4.0 mm, is coated with gold by means of the techniques described above (most simply by wrapping the thulium piece). Most conveniently, one of the open bases of tube 11 is closed with a plug 12a, and then the coated thulium rod 12 is introduced into the titanium tube through its open base, such that a thulium 169 rod 12t with a gold coating 12g provided thereon is affixed within the interior space 11i of the tube. It should be noted that the gold coating is provided on the lateral surface of the thulium 169 rod 12t and on its two bases. After placing the coated thulium section 12 in tube 11, the open base of tube 11 is sealed by a plug 12b. The plugs 12a, 12b are preferably made from one of the materials indicated above for tube 11, preferably from titanium. The diameter of the plugs 12a, 12b is adapted to snugly fit into tube 11, and their length is typically about 0.5 mm. After sealing tube 11 with plug 12b and following laser welding, the radiation source may be activated in a nuclear reactor by means of a neutron flux as described herein below. For considerations of safety, the seed 10 is delivered within a quartz ampoule to the nuclear reactor.

Alternative geometry of the radiation source 22 used in a brachytherapy seed 10 is shown in Figure 2, where the thulium section 22t has the shape of rectangular parallelpiped having the following preferred dimensions:

length between 1.0 and 4.0 mm, more preferably about 4 mm; width between 0.2 and 0.6mm, more preferably about 0.6mm; height between 0.2 and 0.4 mm. The rectangular parallelpiped may be obtained either by using one thulium 169 sheet with the desired shape and size, or by stacking together a plurality of thin thulium 169 sheets 22y, i.e., each sheet being 0.1 mm thick. Thulium sheets are commercially available (Goodfellow, England), and may be readily cut to the desire dimensions using a suitable punch. The thulium section is coated with gold 22g, introduced into the titanium tube 11 and sealed as described above with reference to Figure 1. The sealed capsule may be delivered to a nuclear reactor for activation.

Figure 3 illustrates an embodiment of the invention which is similar to the embodiment shown in Figure 1, with the thulium section having a cylindrical symmetry. However, in this case the thulium section 12t is provided by a hollow rod, namely, a tube-like member defined by a lateral surface of a cylinder, rather than the solid rod exemplified to Figure 1. The thickness of the walls of the thulium tube according to this embodiment is in the range of 0.1-0.2 mm (the thulium tube may be prepared by folding a thulium sheet into a tubular body). The thulium tube can be coated with gold on its outer surface, 12g, or the gold plating is applied externally onto the outer surface of the titanium capsule, rather than being internally placed. Following sealing as described above, the capsule may be delivered to a nuclear reactor for activation.

As mentioned above, the sealed capsule, with the natural thulium (Tm169) and the radiation emission modifying metal coating, is subjected to neutron activation at a nuclear reactor. The number of Tm170 atoms produced by the neutron activation is relative to the number of Tm169 atoms being activated. The number of Tm170 atoms obtained in the neutron activation equals the number of Tm169 atoms irradiated, times the neutron fluence times the activation cross-section. (Neutron fluence =neutron flux times activation time). The activity (number of disintegrations per second) of the Tm170 source, is given by the number of Tm170 atoms times the decay constant. Table 1 shows the activation time, in hours, at flux of $10^{13}$ n/s.cm$^2$, for three different geometries of the thulium section and four different activities. The number of Tm170 atoms which will provide the respect activity is also given in table 1. It is preferred to activate the thulium at a neutron flux of the order of $10^{13}$n/cm$^2$s so that the activation time is not too long (up to 24 hours). The activation time must be such that the desired activity is obtained, as explained above. This time in hours is given in table 1. Having obtained the required activity, the seeds must stay for about two weeks under shield for the heavy metal activity to decay.

Table 1: Time for activation to the given activity, in hours, at flux of $10^{13}$ n/s•cm$^2$

| Activity ----▶ Shape ▼ | 0.5 mCi | 1.0 mCi | 1.5 mCi | 2.0 mCi |
|---|---|---|---|---|
| Cylinder | 2.2 | 4.4 | 6.6 | 8.8 |
| 0.4 mm thick rectangular parallelpiped | 2.59 | 5.17 | 7.76 | 10.35 |
| 0.2 mm thick rectangular parallelpiped | 5.18 | 10.35 | 15.5 | 20.7 |
| Number of Tm170 Atoms for Activity | $2.95 \times 10^{14}$ | $5.9 \times 10^{14}$ | $8.86 \times 10^{14}$ | $1.18 \times 10^{15}$ |

The Tm170-containg seed obtained following the activation described above emits both beta and gamma rays and therefore it can be used for treatment of small tumors as well as large tumors. The beta rays may be used for treating small tumors, while the gamma photons are more suitable for treating large tumors. According to a preferred embodiment of the invention, the radiation emission modifying metal completely surrounds the thulium and has a substantially uniform thickness. By the term "substantially uniform thickness" is meant that the tolerance in the thickness of the coating is not more than ±10%. Thus, the brachytherapy seed of the invention is essentially isotropic, namely, the radiation emitted therefrom has essentially the same intensity regardless of the direction of measurement. The intensity of the isotropic beta radiation generated by the seed is more than 10%, and preferably in the range between 10% and 30%, and more preferably in the range between 20 and 30%, relative to the intensity of the beta radiation emitted by a corresponding non-coated thulium 170. Changes in the emission of beta radiation can be determined by applying a radiation detector, either a thin window ion chamber or a scintillator.

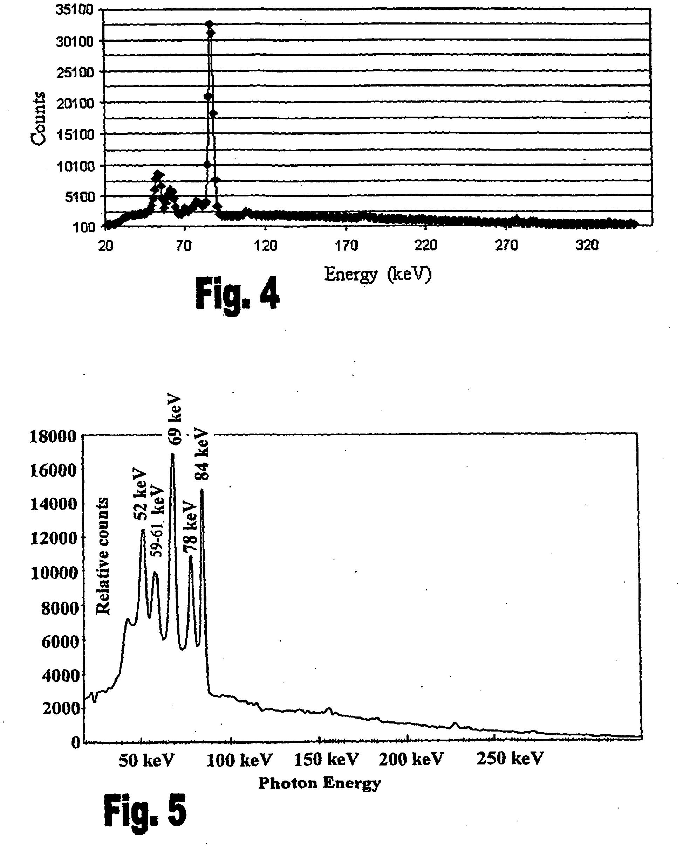

The range of the 970 keV beta rays, which comprises 76% of the beta rays emitted from the Tm170, is about 3 mm in water. However, the presence of a metal (gold) coating of thickness of between few atomic layers (e.g., few Angstroms) to about 0.15 mm favorably modifies the radiation emission profile of the source, in cases wherein the beta rays are not wanted or their intensity should be lowered. Specifically, in case of a thick heavy metal layer (e.g., ~0.15mm), all beta radiation of energy of up to 1 MeV is blocked. The intensity of the photon radiation in this case is increased due to the bremsstrahlung effect in the heavy metal coating layers in which a part of the beta radiation turns into photons. The radiation emission profiles of the modified source according to the present invention will now be discussed in more detail with reference to Figure 4 to 6. In these figures the abscissa is the energy of the emitted photon and the ordinate is the intensity, recorded by multichannel analyzer. Photon spectrum measurements may be carried out using a conventional gamma spectrometry system, which includes: a gamma solid state detector, either HPGe or Si(Li), a high voltage supply, preamplifier, amplifier and a multichannel analyzer. Changes in the emission of beta radiation can be determined by applying a radiation detector, either a thin window ion chamber or a scintillator.

The radiation emitted from Tm 170 is composed of beta radiation (electrons) and gamma radiation (photons). 76% of the beta radiation is of energy $E_{max}$=968 keV and 24% of it is of energy $E_{max}$=884 keV. Fig. 4 shows the characteristic photon spectrum of Tm 170 (non-coated). As seen in Fig. 4, the photon spectrum has a main peak at 84 keV energy level and other smaller peaks, in the energy range 50-60 keV. More specifically, there are three photon energies emitted from Tm170, the first is composed of gamma rays having a 84 keV energy level emitted from excited Y170 atom formed upon the beta decay of Tm170, and the other two are x-rays at about 50-60 keV energy level, emitted following the rearrangement of the Y170 electrons, after the interaction of some of the photons emitted from the nucleus, with the atomic electrons. The ratio of gamma to x-ray emission intensity is typically about 3:1.

When the beta rays emitted from the Tm 170 enter the heavy metal layer (e.g., gold) covering the thulium, most of them are stopped by collision with the electrons of the heavy metal atoms. This is the main beta removal effect caused by the heavy metal. A small fraction, 2-5% as shown in table 2 below, undergo the effect known as braking radiation (Bremsstrahlung in German) due to deceleration of these electrons in the electric field of the heavy metal electrons or the heavy metal nuclei. The energy of those beta rays is turned into photons of continuous spectrum, mostly in the lower energy range. The fraction of the beta ray turning into photons by braking radiation is proportional to the beta energy times the atomic number of the stopping material. The fraction of the beta intensity turning into photons and the photons energy obtained for each beta ray are shown in table 2 for three possible heavy metals: platinum, gold or lead. The columns marked by the % symbol in table 2 indicate the percent of the beta rays turned into photons in the braking radiation process, for each beta energy. The columns entitled by "Energy" give the photon energy obtained from the beta rays, for each beta energy. The columns entitled by "76%" and "24%" give the actual photon energy obtained, from each beta energy. The last column is the sum of the two, i.e. total photon energy obtained from the beta rays by braking radiation.

**TABLE 2** - Photon fractions added to the spectrum due to Braking Radiation

| Metal | Atomic Number | d gm/cm$^3$ | $E_{max}$= 968 keV | | | $E_{max}$= 884 keV | | | Total max energy Per Disintegration |
| --- | --- | --- | --- | --- | --- | --- | --- | --- | --- |
| | | | % | E [keV] | 76% [keV] | % | E [keV] | 24% [keV] | |
| Pt | 78 | 21.45 | 2.64 | 25.6 | 19.44 | 2.41 | 21.33 | 5.12 | 24.56 keV |
| Au | 79 | 19.3 | 2.68 | 25.9 | 19.69 | 2.44 | 21.6 | 5.19 | 24.88 keV |
| Pb | 82 | 11.35 | 2.78 | 26.9 | 20.44 | 2.45 | 22.43 | 5.38 | 25.82 keV |

It should be noted that the activation of the seed may be carried out either prior or subsequent to the incorporation of the radiation emission modifying metal within the seed. In the event that the coating is applied in the seed before the activation, namely, a titanium casing which comprises Tm 169 and a gold or platinum layer is activated by neutrons to get the radioactive isotope Tm 170, then the coating material is activated too and a radioactive isotope thereof is formed. The activation of the titanium is negligible. These radioactive isotopes are short lived: T½Pt195=4 days, T½Au198=65 hours, T½Pb209=3.3 hours. The respective activation cross sections are: σPt=10 b, σAu=98.8 b and σPb=0.17 b. The activity of these isotopes is undesired in the radiation source, therefore, after being activated the radiation source should stay in the reactor area for about two weeks cooling period for these isotopes to decay. The two weeks decay time has a minor effect on the Tm 170 activity; it decays to 93% of its activity at the end of the activation.

The heavy metal coating (such as Au, Pt, Pb, Bi, Tl or amalgam of Hg, with gold being especially preferred) surrounds the thulium core in the seed of the present invention and forms part of the radiation source. Although the heavy metal coating is not radioactive, it emits radiation as a result of absorbing the radiation from the Tm 170 core. The heavy metal coating applied over the radiation source reduces, or stops completely (depends on its thickness), the beta rays emitted from the Tm 170 core, from leaving the radiation source into the tumor. A part of the beta rays absorbed by the heavy metal coating the Tm turns into photons as braking radiation. An additional photon source is created due to x-ray emitted from the heavy metal coating, this x rays emission is typical to those metals. These x rays energies are in the range of 66-84 keV respective to the metal emitting it.

Thus, the metal coating the Tm 170 core of the radiation source changes the radiation emitted therefrom in a number of ways. The effect it has on the beta rays is the most drastic because this thin coating either eliminates the beta rays emission completely or reduces it to a desirable degree. The thicker the coating layer the weaker is the beta intensity.

The second effect of the heavy metal coating layer on the radiation emitted from the radiation source is the addition of a continuous x ray spectrum, to the photon emitted from the radiation source. Stopping the beta radiation by the heavy metal causes the emission of braking radiation. This is a continuous photon spectrum of energy from very low (1-3 keV) to the highest energy of the beta rays-Emax emitted from the Tm 170, as can be seen in figure 5.

The third effect of the heavy metal coating on the photon spectrum emitted from the radiation source is the additional peaks in the spectrum, related to x rays emitted from said metal coating. The source of these x rays is the photoelectric effect caused by the 84 keV photons emitted from the Tm 170. The main x ray peaks are at energy levels: 67, 69, 78 and 80.1 for gold; 65, 67, 75.7 and 77.8 for platinum; and 72.8, 75, 84.8 and 87.3 for lead. The main gold peaks are seen in figure 5. The photoelectric effect caused by the 84 keV photons lowers this peak in the spectrum as can be seen in figure 5. (The ratio between the 84 keV peak and the 52.4 keV peak has been reduced compared to that seen in Fig. 4; according to the modified source of the present invention, the ratio between said peaks is less than 2.5:

1). This is fourth spectrum change due to the heavy metal coating the TM 170.

To sum up, the changes in the photon spectrum caused by the heavy metal layer are: additional low energy photons, reduction in the 84 keV peak and addition of x rays peaks corresponding to the metal coating the thulium radiation source. The change in the beta spectrum: lowering its intensity or complete removal.

The addition of the low energy photons to the spectrum increases the efficiency of the radiation source at small distance. Although lowering the 84 keV photons peak reduces the number of photons that can reach large distance from the source, this is sufficiently compensated by the addition of the higher energy x rays emitted by the heavy metal and by the higher photons in the braking radiation.

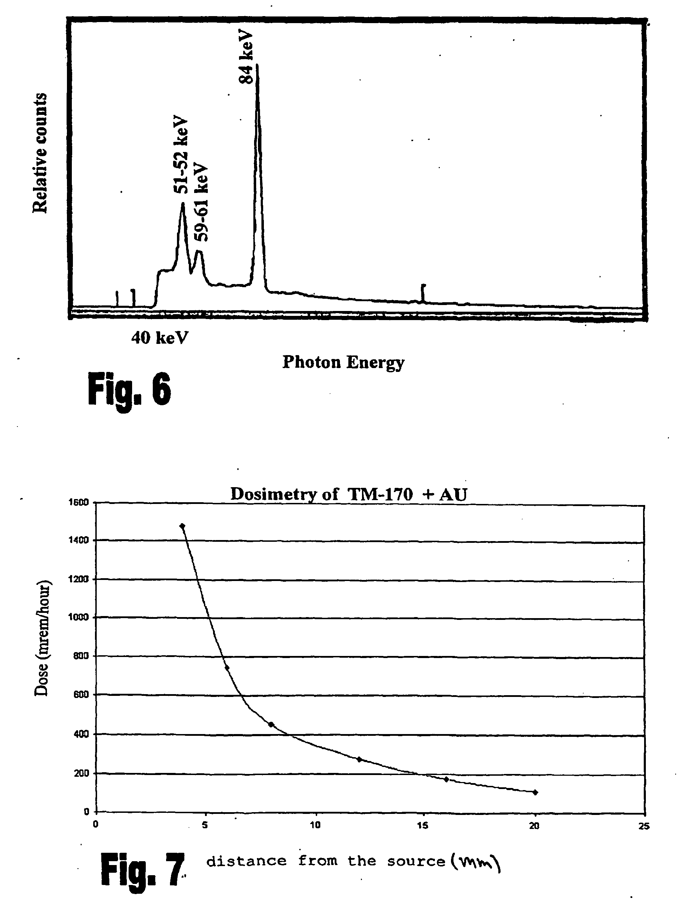

Fig. 6 (comparative) shows the photon spectrum of a Tm170 radiation source in which the radioactive section was not coated by gold but encased in a titanium seed having wall thickness of about 0.05mm. The continuous spectrum beyond the 84 keV peak is due to braking radiation in the titanium.

In Figure 7, the radiation dose associated with the gamma emission generated by 2 mCi Tm 170 seed, wherein the thulium is coated with 0.1 mm gold foil, is plotted as a function of distance from the source. The radiation dose was measured with LiF(Mg,Ti) TLD in Lucite (PMMA) phantom. The units of the ordinate are millirem per hour.

The seed according to the present invention is deliverable to the tumor site by means of commonly applied applicators (e.g., catheters, needles) in accordance with low dose rate brachytherapy protocols. For example, in the case of prostatic cancer, one possible technique involves loading the seeds into the cannula of a needle-like insertion device. Improved techniques for implanting brachytherapy seeds, which may be practiced according to the present invention are disclosed, for example, in US 6,036,632, US 6,267,718 and US 6,311,084. Further references describing brachytherapy protocols are the following books:

Radiation Aspects of Brachytherapy For Prostate Cancer. ELSEVIER, Jul 2006.

Brachytherapy Oncology Update 1984, B.S. Hilaris and D. Nori, ed. Memorial Sloan-Kettering Cancer Center, 1275 York Avenue, New York, NY. 1984.

The LDR seed of the present invention may be used in brachytherapy as follows - implanting into or adjacent a tumor site of a patient a plurality of low dose rate thulium 170 brachytherapy seeds, wherein the thulium brachytherapy seed is provided with a coating of a radiation emission modifying metal, the activity of each of said seeds being less than 3 millicurie, whereby beta radiation and gamma radiation, which are preferably isotropic, are delivered to said tumor. The tumors that can be treated with the seeds of the present invention include prostate, breast, various gynecological and head and neck tumors. The number of seeds to be implanted is determined according to the type and size of the tumor, and may vary in the range between 20 and 100.

Brief Description of the Drawings

The present invention is illustrated by way of example in the accompanying drawings, in which similar references consistently indicate similar elements and in which:

- **-** Fig. 1 illustrates one embodiment of the LDR brachytherapy seed of the invention;
- **-** Fig. 2 illustrates another embodiment of the LDR brachytherapy seed of the invention;
- **-** Fig. 3 illustrates another embodiment of the LDR brachytherapy seed of the invention.
- **-** Fig. 4 (comparative) shows the characteristic photon spectrum emitted by Tm-170;
- **-** Fig. 5 shows a photon energy spectrum measured for a Tm170 radiation source of the invention having a 0.1mm gold layer coating;
- **-** Fig. 6 (comparative) shows a photon spectrum of a Tm170 radiation source encased in a titanium capsule having wall thickness of about 0.05mm; and
- **-** Fig. 7 shows the radiation dose from a radiation source comprising thulium 170 coated with 0.1 mm gold foil.

It should be noted that the embodiments exemplified in the Figures are not intended to be in scale and are in diagram form to facilitate ease of understanding and description.

## **Examples**

### **Example 1**

Preparation of a seed comprising thulium 170 wrapped with a gold foil in a titanium canister

This example illustrates the preparation of the seed shown in Figure 1.

Thulium 169 wire of 0.6 mm diameter was cut to obtain a section having a desired length (in the range between 1.0 mm and 5.0 mm). The thulium piece was then wrapped with a gold foil having thickness of about 0.1mm (Goodfellow, England).

One end of a titanium tube (0.8 mm outer diameter, 0.7 inner diameter) was closed with a titanium plug. The plug is made of a titanium wire of diameter 0.7 mm (the inner diameter of the tube). A slice of 0.5 mm long of this wire was plugged into the titanium tube on one end and welded with a laser beam. The length of the titanium tube used depends on the length of the thulium piece + 0.5 mm for a plug insertion on each end.

The gold coated thulium was inserted into the titanium tube through the open end. The second end of the tube was also plugged and sealed by welding. The titanium tube is now ready to be activated in order to convert the Tm-169 into Tm-170. The Activation time and flux level, which determine the source activity, were described hereinabove.

## Example 2

Preparation of a titanium seed comprising thulium 170 plated with gold

This example illustrates the preparation of the seed shown in Figure 1, wherein the thulium wire was plated with gold, applying a spattering technique. E5100 Polaron system was used for this purpose.

A gold foil, less than 1.0 mm thick on aluminum base was used as the gold source. The gold foil has a shape of a flat ring 10 cm diameter, 1 cm thick. This gold ring and the thulium seeds (thulium wire 0.6 mm diameter, 5 mm long) to be plated were placed in a vacuum chamber. The air was pumped out and argon gas was let into the chamber to a pressure of 0.1 to 0.2 Torr. The argon is kept flowing into the chamber during the gold plating to keep the pressure constant. A voltage of the order of 1-2 kV was applied between the gold electrode and the thulium wire pieces. The distance between the gold electrode and the thulium wire was about 50 mm. Ion current of about 20 mA flew between the gold electrode and the thulium wire, causing gold spattering from the gold electrode. Under such conditions, every five minutes the thickness of the gold layer increases in 75 Å. The growth of the gold coating was allowed to continue for 60 minutes. The gold-coated thulium is inserted into a titanium capsule and sealed as described in Example 1. The sealed capsule is delivered to a nuclear reactor to be activated as described hereinabove.

## Example 3

Preparation of a seed comprising thulium 170

This example illustrates the preparation of the seed shown in Figure 1. However, the gold coating was applied externally onto the titanium canister subsequent to the activation of the seed in a nuclear reactor.

Thulium 169 wire of 0.6 mm diameter was cut to obtain a section having a desired length (in the range between 1.0 mm and 5.0 mm). One end of a titanium tube (0.8 mm outer diameter, 0.7 inner diameter) was closed with a titanium plug. The plug is made of a titanium wire of diameter 0.7 mm (the inner diameter of the tube). A slice of 0.5 mm long of this wire was plugged into the titanium tube on one end and welded with a laser beam. The length of the titanium tube used depends on the length of the thulium piece + 0.5 mm for a plug insertion on each end. The thulium was inserted into the titanium tube through the open end. The second end of the tube was also plugged and sealed by welding. The titanium tube was transferred for activation in a nuclear reactor, in order to convert the Tm-169 into Tm-170. For considerations of safety, the seed is delivered within a quartz ampoule to the nuclear reactor. The seeds were activated at a neutron flux of $10^{13}$ n/s•cm$^2$ for 12 hours and were subsequently coated with a gold foil.

## Claims

1. A brachytherapy seed (10) comprising thulium-170 (12t) placed within a casing (11), **characterized in that** said brachytherapy seed is a low dose rate seed having activity of less than 3 millicurie and at least one layer of a radiation emission modifying metal (12g), said layer being provided either internally within the casing or on the outer surface thereof, wherein:

   (i) the level of the beta radiation emitted is reduced in comparison with that generated by thulium 170;
   (ii) the photon emission spectrum of the source displays a lower ratio between the intensities of the characteristic peaks at 84 keV and 52 keV energy levels, relative to the ratio observed in the corresponding spectrum of thulium 170;
   (iii) the photon emission spectrum of said source exhibits one or more peaks in the region between 65 keV and 84 keV energy levels indicative of photons emitted by the layer(s) of the radiation emission modifying metal; and

(iv) the photon emission spectrum of said source comprises in addition to the discrete peaks also a continuous region indicative of the emission of braking radiation associated with the layer(s) of the radiation emission modifying metal.

2. A brachytherapy seed according to claim 1, wherein the radiation emission modifying metal is provided as a coating surrounding the thulium, said coating having a substantially uniform thickness.

3. A brachytherapy seed according to claim 2 which emits isotropic beta radiation, the intensity of said radiation being in the range between 10% and 30% relative to the intensity of the beta radiation emitted by a corresponding non-coated thulium 170.

4. A brachytherapy seed according to claim 2, wherein the thulium is provided as a solid cylinder, a rectangular parallelpiped or a tube-like member defined by a lateral surface of a cylinder.

5. A brachytherapy seed according to claim 4, where the thulium is provided by a tube-like member defined by a lateral surface of a cylinder, the thickness of the walls of said tube-like member being in the range between 0.1 and 0.2 mm.

6. A brachytherapy seed according to any one of the preceding claims, wherein the casing is made of titanium and the radiation emission modifying metal is provided as a gold coating or platinum coating.

7. A brachytherapy seed according to claim 6, wherein the coating comprises gold, such that the ratio between the intensities of the characteristic peaks of thulium 170 at 84 keV and 52 keV energy levels is less than 2.5:1, and wherein said seed further exhibits at least one X-ray peaks at energy levels of about 65 to 84 keV.

**Patentansprüche**

1. Brachytherapie-Seed (10), der Thulium-170 (12t) umfasst, das in ein Gehäuse (11) eingesetzt ist, **dadurch gekennzeichnet, dass** der Brachytherapie-Seed ein Seed niedriger Dosisrate ist, der eine Aktivität von weniger als 3 Millicurie und wenigstens eine Schicht aus Strahlungsemission modifizierendem Metall (12) hat, und wobei die Schicht entweder im Inneren des Gehäuses oder an seiner Außenfläche vorhanden ist, wobei:

(I) der Pegel der emittierten β-Strahlung im Vergleich zu dem durch Thulium 170 erzeugten reduziert wird;
(II) das Photonenemissions-Spektrum der Quelle relativ zu dem Verhältnis, das in dem entsprechenden Spektrum von Thulium 170 zu beobachten ist, ein niedrigeres Verhältnis zwischen den Intensitäten der Kennlinien-Spitzen bei Energiepegeln von 84 keV und 52 keV aufweist;
(III) das Photonenemissions-Spektrum eine oder mehrere Spitzen in dem Bereich zwischen Energiepegeln von 65 keV und 84 keV aufweist, die Photonen anzeigen, die durch die Schicht/en des Strahlungsemission modifizierenden Metalls emittiert werden; und
(IV) das Photonenemissionsspektrum der Quelle zusätzlich zu den diskreten Spitzen auch einen kontinuierlichen Bereich umfasst, der die Emission von Bremsstrahlung anzeigt, die mit der/den Schicht/en des Strahlungsemission modifizierenden Metalls zusammenhängt.

2. Brachytherapie-Seed nach Anspruch 1, wobei das Strahlungsemission modifizierende Metall als eine Beschichtung vorhanden ist, die das Thulium umgibt, und die Beschichtung eine im Wesentlichen gleichmäßige Dicke hat.

3. Brachytherapie-Seed nach Anspruch 2, der isotropische β-Strahlung emittiert, wobei die Intensität der Strahlung relativ zu der Intensität der durch ein entsprechendes nicht beschichtetes Thulium 170 emittierten β-Strahlung im Bereich zwischen 10 % und 30 % liegt.

4. Brachytherapie-Seed nach Anspruch 2, wobei das Thulium als ein massiver Zylinder, ein rechteckiges Parallelepiped oder ein röhrenartiges Element vorliegt, das durch eine Seitenfläche eines Zylinders gebildet wird.

5. Brachytherapie-Seed nach Anspruch 4, wobei das Thulium als ein röhrenartiges Element vorliegt, das durch eine Seitenfläche eines Zylinders gebildet wird, und die Dicke der Wände des röhrenartigen Elementes in dem Bereich zwischen 0,1 und 0,2 mm liegt.

6. Brachytherapie-Seed nach einem der vorangehenden Ansprüche, wobei das Gehäuse aus Titan besteht und das Strahlungsemission modifizierende Metall als eine Goldbeschichtung oder Platinbeschichtung vorliegt.

**7.** Brachytherapie-Seed nach Anspruch 6, wobei die Beschichtung Gold umfasst, so dass das Verhältnis zwischen den Intensitäten der Kennlinien-Spitzen von Thulium 170 bei Energiepegeln von 84 keV und 52 keV weniger als 2,5:1 beträgt, und der Seed des Weiteren wenigstens Röntgenstrahl-Spitzen bei Energiepegeln von ungefähr 65 bis 84 keV aufweist.

**Revendications**

**1.** Grain de curiethérapie (10) comprenant du thulium-170 (12t) placé dans un boîtier (11), **caractérisé en ce que** ledit grain de curiethérapie est un grain à faible dose ayant une activité inférieure à 3 millicuries et au moins une couche d'un métal modifiant les émissions de radiations (12g), ladite couche étant ménagée soit intérieurement dans le boîtier ou sur la surface extérieure de celui-ci, dans lequel :

(i) le niveau de la radiation bêta émise est réduit par rapport à celle générée par le thulium 170 ;
(ii) le spectre d'émission de photons de la source affiche un rapport entre les intensités des pics caractéristiques à des niveaux d'énergie de 84 keV et de 52 keV inférieur, relativement au rapport observé dans le spectre correspondant de thulium 170 ;
(iii) le spectre d'émission de photons de ladite source présente un ou plusieurs pics dans la zone comprise entre 65 keV et 84 keV des niveaux d'énergie indicatifs de photons, émis par la (les) couche(s) du métal modifiant l'émission de radiation ; et
(iv) le spectre d'émission de photons de ladite source comprend, en plus des pics discrets, également une zone continue indicative de l'émission de radiations de freinage associées à la (aux) couche(s) de métal de modification d'émission de radiation.

**2.** Grain de curiethérapie selon la revendication 1, dans lequel le métal modificateur de l'émission de radiation est fourni comme un revêtement entourant le thulium, ledit revêtement ayant une épaisseur sensiblement uniforme.

**3.** Grain de curiethérapie selon la revendication 2, qui émet une radiation bêta isotrope, l'intensité de ladite radiation étant dans la gamme de 10 % à 30 % par rapport à l'intensité de la radiation bêta émise par un thulium 170 non revêtu correspondant.

**4.** Grain de curiethérapie, selon la revendication 2, dans lequel le thulium est fourni sous la forme d'un cylindre solide, d'un élément parallélépipédique rectangulaire ou tubulaire défini par une surface latérale d'un cylindre.

**5.** Grain de curiethérapie selon la revendication 4, dans lequel le thulium est fourni par un élément tubulaire défini par une surface latérale d'un cylindre, l'épaisseur des parois dudit élément tubulaire étant dans la gamme comprise entre 0,1 et 0,2 mm.

**6.** Grain de curiethérapie selon l'une quelconque des revendications précédentes, dans lequel le boîtier est réalisé en titane et le métal modifiant les émissions de radiations est fourni sous la forme d'un revêtement en or ou d'un revêtement en platine.

**7.** Grain de curiethérapie selon la revendication 6, dans lequel le revêtement comprend de l'or, de sorte que le rapport entre les intensités des pics caractéristiques du thulium 170, à des niveaux d'énergie de 84 keV et 52 keV, est inférieur à 2,5 : 1, et dans lequel ledit grain présente en outre au moins un pic de rayons X à des niveaux d'énergie d'environ 65 à 84 keV.

**Fig. 1**

**Fig. 2**

**Fig. 3**

**Fig. 4**

**Fig. 5**

Fig. 6

Dosimetry of TM-170 + AU

Fig. 7  distance from the source (mm)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 03063757 A
- EP 1529554 A
- WO 2008041230 A
- US 2006219956 A

- US 6036632 A
- US 6267718 B
- US 6311084 B

**Non-patent literature cited in the description**

- Radiation Aspects of Brachytherapy For Prostate Cancer. ELSEVIER, July 2006

- Brachytherapy Oncology Update. Memorial Sloan-Kettering Cancer Center. 1984, 1275